# FASCICULE DE BREVET EUROPEEN

(11) **EP 0 815 849 B1**
(45) Date de publication et mention de la délivrance du brevet: **16.06.1999**
(21) Numéro de dépôt: 97401285.8
(22) Date de dépôt: 06.06.1997
(51) Int. Cl.: A61K 7/48, A61K 7/06

(54) **Utilisation d'oligomères comme plastifiants d'un polymères filmogène dans et pour la préparation de compositions cosmétiques contenant ce polymére filmogène, compositions cosmétiques ou dermatologiques**
Verwendung von Oligomeren als Weichmacher eines filmbildenden Polymers in der und zur Herstellung von kosmetischen Zusammensetzungen die dieses filmbildendes Polymer enthalten, kosmetische und dermatologische Zusammensetzungen
Use of oligomers as plasticizers for a filmforming polymer in and for the preparation of cosmetical compositions containing this filmforming polymer, cosmetical or dermatological compositions

(30) Priorité: 28.06.1996 FR 9608114
(43) Date de publication de la demande: 07.01.1998
(73) Titulaire: L'OREAL, 75008 Paris (FR)
(72) Inventeur: Lion, Bertrand, 93190 Livry-Gargan (FR); Dupuis, Christine, 75018 Paris (FR)
(74) Mandataire: Miszputen, Laurent

(56) Documents cités:
- EP-A- 0 602 513
- DE-A- 2 928 190
- FR-A- 2 239 480

## Description

La présente invention a trait à l'utilisation de certains oligomères comme agent plastifiant d'un polymère filmogène dans et pour la préparation de compositions cosmétiques ou dermatologiques contenant au moins ce polymère filmogène ainsi que les formulations topiques contenant ces agents plastifiants notamment les compositions capillaires pour le coiffage, pour le maintien et/ou la fixation de la chevelure et les compositions de maquillage.

Dans les compositions cosmétiques à base de polymères filmogènes, en particulier les compositions capillaires pour le coiffage ou le maintien de la chevelure comme les laques aérosols, on utilise en général des agents plastifiants en association avec les polymères filmogènes afin d'abaisser la température de transition vitreuse de ces derniers et de moduler, selon l'application choisie, les propriétés mécaniques des films résultant desdits polymères telles que la flexibilité. Les plastifiants généralement utilisés sont choisis parmi des solvants non-volatils usuels du polymère filmogène employé. Ceux-ci restent piégés dans le dépôt du polymère filmogène après séchage. On connaît également certains polymères plastifiants du type polyéther d'éthylène glycol ou de propylène glycol tels que les produits DOWANOL de la société DOW CHEMICAL. On connaît aussi le document FR-2 239 480 qui décrit un procédé de préparation de polymères téléchéliques et les produits ainsi obtenus.

La Demanderesse a découvert de façon surprenante une nouvelle famille d'agents plastifiants pour les polymères filmogènes habituellement utilisés en cosmétique, constituée d'oligomères particuliers que l'on définira plus loin en détail. Ces oligomères conduisent à une meilleure efficacité dans la plastification des polymères filmogènes par rapport aux plastifiants classiques.

Les oligomères conformes à l'invention, lorsqu'ils sont utilisés dans des produits de coiffage à base de polymères filmogènes tels que des laques pour la fixation des cheveux, permettent notamment d'obtenir, dans de plus faibles quantités, un pouvoir laquant équivalent à celui obtenu avec des plastifiants classiques avec des quantités plus élevées, tout en améliorant de manière significative les propriétés cosmétiques des cheveux telles que le démêlage et le toucher après application.

Les oligomères conformes à l'invention permettent également lorsqu'ils sont utilisés dans des produits de maquillage des cils ou des sourcils tels que les mascaras ou les eye-liners, grâce à leurs remarquables propriétés de plastification des polymères filmogènes généralement utilisés dans cette application, de favoriser l'étalement du dépôt ainsi que d'obtenir une bonne flexibilité du dépôt dans des quantités plus faibles que celles généralement utilisées avec des plastifiants classiques.

Les oligomères conformes à l'invention permettent aussi, lorsqu'ils sont utilisés dans des produits de maquillage des ongles tels que les vernis à ongles, grâce à leurs remarquables propriétés de plastification des polymères filmogènes généralement utilisés dans cette application, d'améliorer la brillance dans des quantités plus faibles que celles généralement utilisées avec des plastifiants classiques. De plus, contrairement aux plastifiants classiques, ils ne conduisent pas à des phénomènes de migration en surface ni à des phénomènes de perte par évaporation au cours du temps.

L'objet principal de l'invention consiste en l'utilisation d'au moins un oligomère particulier que l'on définira plus loin comme agent plastifiant d'un polymère filmogène dans et pour la préparation de compositions cosmétiques ou dermatologiques contenant au moins ce polymère filmogène; ces compositions ne contenant pas de polymère filmogène du type nitrocellulose.

Un autre objet de l'invention consiste en des compositions cosmétiques ou dermatologiques contenant au moins un polymère filmogène et au moins un agent plastifiant dudit polymère choisis parmi les oligomères particuliers qui seront définis par la suite.

D'autres objets apparaitront à la lumière de la description et des exemples qui suivent.

On entend par agent plastifiant d'un polymère filmogène donné, toute substance compatible avec ledit polymère et susceptible de se combiner avec ou s'insérer dans ledit polymère de telle sorte que le mélange obtenu présente une température de transition vitreuse et une température de ramollissement inférieures à celles du polymère filmogène et lui confère ainsi une meilleure flexibilité.

Les oligomères de l'invention sont essentiellement caractérisés par le fait qu'ils sont choisis parmi les homopolymères ou copolymères de monomère(s) à insaturation éthylénique et les polycondensats, ayant un poids moléculaire moyen mesuré en sommet de pic par chromatographie par exclusion stérique, allant de 400 à 10000 et ayant une température de transition vitreuse allant de - 80 à 10°C.

Leur poids moléculaire moyen mesuré en sommet de pic par chromatographie par exclusion stérique varie plus particulièrement de 500 à 5000.

Leur température de transition vitreuse varie plus particulièrement de -70 à 5°C.

Les oligomères selon l'invention constitués de polymère d'au moins un monomère à insaturation éthylénique sont choisis par exemple parmi les homopolymères ou copolymères acryliques, méthacryliques, allyliques, méthallyliques ou vinyliques. Ils peuvent être anioniques, cationiques, amphotères ou non-ioniques.

Ces monomères à insaturation éthylénique peuvent être choisis notamment dans le groupe constitué par :
- les esters ou les amides acryliques ou méthacryliques obtenus à partir d'alcools aliphatiques, linéaires, ramifiés ou cycliques et/ou d'alcools aromatiques, de préférence en C₁-C₃₀ tel que le (méth)acrylate de méthyle, le (méth)acrylate d'éthyle, le (méth)acrylate de propyle, le (méth)acrylate de butyle, le (méth)acrylate d'isobutyle, le (méth)acrylate de tertio-butyle, le tertio-butyl acrylamide ;
- les esters ou amides vinyliques, allyliques ou méthallyliques obtenus à partir d'alcools aliphatiques, linéaires, ramifiés ou cycliques et/ou d'alcools aromatiques, de préférence en C₁-C₃₀, tels que l'acétate de vinyle, le propionate de vinyle, le benzoate de vinyle, le tertio-butyl benzoate de vinyle ;
- les oléfines tels que l'éthylène, le propylène, le styrène ou le styrène substitué ;
- les monomères acryliques ou vinyliques halogénés ou perhalogénés et plus particulièrement fluorés ou perfluorés ;
- leurs mélanges.

On peut citer parmi les monomères anioniques à insaturation éthylénique :
- les monomères comportant au moins une fonction acide, sous forme libre ou bien sous forme partiellement ou totalement neutralisée tels que les mono acides carboxyliques comme les acides acrylique, méthacrylique, crotonique ; les diacides carboxyliques ou les anhydrides d'acide ainsi que leurs monoesters ou monoamides comme l'anhydride maléique sous forme de diacide, de monoester ou monoamide, l'acide itaconique ;
- les monomères comportant au moins une fonction acide sulfonique, sous forme libre ou bien sous forme partiellement ou totalement neutralisée tels que l'acide vinyl-ou styrène sulfonique, l'acide acrylamido-2 méthylpropane-2 sulfonique ;
- les monomères comportant au moins une fonction acide phosphorique ou phosphonique, sous forme libre ou bien sous forme partiellement ou totalement neutralisée.

Ces monomères anioniques sont de préférence partiellement ou totalement neutralisés par un composé monobasique telle qu'une base minérale comme la soude ou la potasse, ou un aminoalcool par exemple pris dans le groupe constitué par l'amino-2 méthyl-2 propanol-1 (AMP), la triéthanolamine, la triisopropanolamine (TIPA), la monoéthanolamine, la tri[(hydroxy-2) propyl-1 amine, l'amino-2 méthyl-2 propanediol-1,3 (AMPD), l'amino-2 hydroxyméthyl-2 propanediol-1,3.

On peut citer parmi les monomères cationiques à insaturation éthylénique :
- les monomères comportant une fonction amine sous forme libre ou bien partiellement ou totalement neutralisée ou bien partiellement ou totalement quaternisée tels que le (méth)acrylate de diméthylaminoéthyle, le diméthylaminoéthyl méthacrylamide, la vinylamine, la vinylpyridine, le chlorure de diallyldiméthylammonium.

Ces monomères cationiques sont de préférence partiellement ou totalement neutralisés par un acide organique minéral ou organique tel que les acides chlorhydrique, acétique, lactique ou glycolique ou bien partiellement ou totalement quaternisés par un halogénure d'alkyle, de cycloalkyle, d'aryle ou un dialkylsulfate (diméthyl- ou diéthylsulfate).

On peut citer parmi les monomères amphotères à insaturation éthylénique
- les carboxybétaïnes ou les sulfobétaïnes obtenues par quaternisation partielle ou totale de monomères à insaturation éthylénique comportant une fonction amine par des sels de sodium d'acide carboxylique à halogénure mobile (chloroacétate de sodium) ou par des sultones cycliques (propane sultone).

Parmi les monomères non-ioniques à insaturation éthylénique, on peut citer :
- les (méth)acrylate d'hydroxyalkyle en C₁-C₃₀ tels que (méth)acrylate de 2-hydroxy éthyle, (méth)acrylate de 2-hydroxy propyle ;
- les acrylamides tels que acrylamide, méthacrylamide, les dialkyl(C₁-C₃₀) (méth) acrylamides ;
- la N-vinylpyrrolidone, la vinylcaprolactam ;
- les (méth)acrylates d'éthylèneglycol, de diéthylèneglycol, de polyéthylèneglycol à extrémité hydroxyle ou éther.

Les oligomères constitués de polymère d'au moins un monomère à insaturation éthylénique, peuvent être obtenus par des procédés de polymérisation radicalaire classiques adaptés pour l'obtention de poids moléculaires faibles. On peut citer les méthodes de polymérisation en solution, en suspension, en émulsion, par précipitation ou en dispersion. Pour favoriser l'obtention de faibles poids moléculaires, on utilise de préférence des quantités importantes d'agents de transfert pour ajuster plus facilement le poids moléculaire. On citera à ce titre, les procédés de télomérisation tels que définis dans l'Encyclopédie 〈〈Comprehensive Polymer Science〉〉, Vol 3, pages 185-194, PERGAMON PRESS, 1989.

Les oligomères du type polycondensat selon l'invention sont choisis par exemple parmi les polyesters, les polyester/amides, les polyamides, les polyuréthanes et les polyurée ou leurs mélanges. Ils peuvent être anioniques, cationiques, amphotères ou non-ioniques.

Les polyesters, les polyester/amides, les polyamides peuvent résulter par exemple de la polycondensation entre diols, diacides carboxyliques, aminoalcools ou diamines pouvant comporter des chaînes grasses latérales, de préférence saturées. Ils peuvent également résulter de la polycondensation sur lui-même d'un composé comportant à la fois un groupe réactif acide carboxylique et un groupe réactif alcool ou bien comportant à la fois un groupe réactif acide carboxylique et un groupe réactif amine. Parmi les monomères présents dans le milieu de polycondensation, on citera également la présence possible d'anhydrides carboxyliques (anhydride phtalique) et de composés ne comportant qu'un seul groupe réactif tels que des monoalcools, des monoamines, des monoacides carboxyliques, de préférence gras. Les monomères de polycondensation mono ou difonctionnels sont de nature aliphatique, linéaire, ramifié ou cyclique ou bien de nature aromatique.

Les polyesters, les polyester/amides, les polyamides sont obtenus selon les méthodes classiques de polycondensation à l'état fondu, en solution ou en dispersion.

Une famille particulièrement préférée d'oligomères conformes à l'invention est constituée de polyesters susceptibles d'être obtenus par polycondensation d'au moins un diacide carboxylique choisi dans le groupe constitué par :
- (i) les composés de formule (1) suivante :

   HOOC-(CH₂)ₘ-COOH (1)

   où m est un nombre entier allant de 2 à 20 ;
- (ii) les composés de formule (2) suivante : 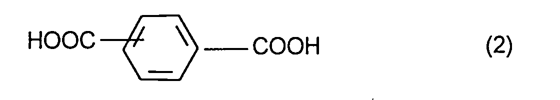 le groupe acide mobile étant de préférence en position ortho, para ou méta et le cycle benzénique pouvant comporter d'autres substituants différents des groupes sulfonates ;
- (iii) leurs mélanges ,
   avec un diol choisi dans le groupe constitué par :
- (a) les composés de formule (3) suivante :

   HO-(CH₂)ₓ-OH (3)

   où x est un nombre entier allant de 2 à 20 ;
- (b) les composés de formule (4) suivante : 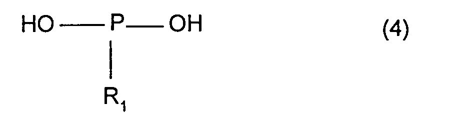 où P est un radical hydrocarboné trivalent aliphatique, linéaire ou ramifié, cycloaliphatique ou aromatique, de préférence un groupe -(CH₂)ₚ-CH- avec p ≥ 1 et plus particulièrement le groupe -CH₂-CH- et R₁ désigne une chaîne latérale hydrocarbonée en C₂-C₃₀ ;
- (c) les composés de formule (5) suivante : 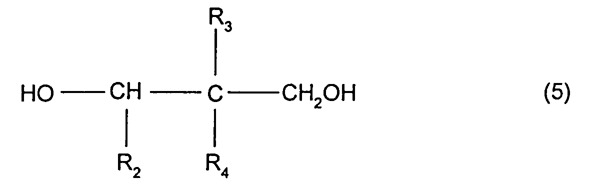 où R₂ désigne un atome d'hydrogène ou un radical alkyle linéaire ou ramifié en C₁-C₃ ; R₃ désigne un atome d'hydrogène ou un radical alkyle linéaire ou ramifié en C₁-C₄ et R₄ désigne un radical alkyle linéaire ou ramifié en C₁-C₄ ;
- (d) le composé de formule (6) suivante : 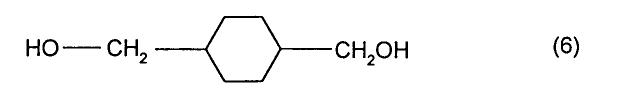
- (e) le composé de formule (7) suivante : 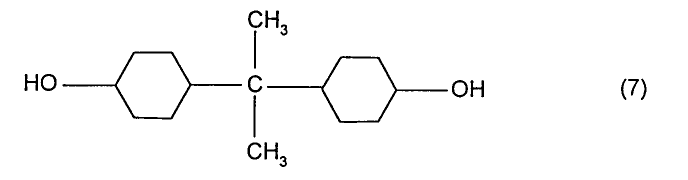
- (f) leurs mélanges.

Les compositions cosmétiques et dermatologiques selon l'invention contiennent donc dans un support cosmétiquement ou dermatologiquement acceptable au moins un polymère filmogène et au moins un oligomère ou télomère tel que défini précédemment comme agent plastifiant dudit polymère filmogène, pour des applications aussi variées que celles rencontrées par exemple dans le domaine du capillaire, du maquillage ou bien encore des soins de la peau, ou de tout autre domaine cosmétique dans lequel l'utilisation d'une substance filmogène est désirable ou recherchée.

Parmi les applications préférentiellement visées par la présente invention, on peut plus particulièrement mentionner :
- le domaine des produits capillaires (lavage, soin ou beauté des cheveux), où les compositions selon l'invention, peuvent se présenter sous forme d'aérosols, de mousse, de shampooings, d'après-shampooings, de lotions ou de gels coiffants ou traitants, laques ou lotions de mise en forme ou de mise en plis ou encore de fixation.
- le domaine des produits de maquillage, en particulier pour le maquillage des ongles, des cils ou des lèvres, où les compositions selon l'invention peuvent se présenter sous forme de vernis à ongle ; de mascaras ou de eye-liners ; de rouges à lèvres.
- dans le domaine des produits de soin de la peau (crèmes, laits, lotions, masques, sérums, produits solaires).

Les agents plastifiants conformes à l'invention sont utilisés de préférence dans des concentrations en matières sèches allant de 0,1 à 80 % en poids, plus préférentiellement de 5 à 40 % et plus particulièrement de 10 à 30 % en poids par rapport au poids total de la composition.

Les polymères filmogènes à plastifier utilisés dans les compositions de l'invention sont ceux habituellement utilisés pour les différentes applications mentionnées ci-dessus. Ils sont choisis par exemple parmi les polymères décrits dans la demande. Ils sont choisis en particulier parmi ceux décrits dans les demandes FR 2.439.798 et FR 2 697 160 et le brevet FR 9213600.

On peut citer en particulier :
- les copolymères acétate de vinyle/acide crotonique/tertio-butyl benzoate de vinyle décrits dans le brevet FR 2 439 798 ;
- les copolymères acide acrylique/acrylate d'éthyle /N-tertiobutylacrylamide tels que le produit vendu sous le nom ULTRAHOLD STRONG par la société BASF ;
- les copolymères N-octylacrylamide/méthacrylate de méthyle/méthacrylate d'hydroxy propyle/acide acrylique/méthacrylate de tert-butyl aminoéthyle tels que le produit vendu sous le nom AMPHOMER LV 71 par la société NATIONAL STARCH ;
- les terpolymères acide acrylique/acrylate d'éthyle/acrylate de tertiobutyle tels que le produit vendu sous le nom LUVIMER 100P par la société BASF.

La concentration en polymère filmogène dans les compositions cosmétiques ou dermatologiques de l'invention est généralement comprise entre 0,1 et 50%, et de préférence entre 1 et 30% en poids par rapport au poids total de la composition. Elle varie selon l'application cosmétique ou dermatologique envisagée.

Le support cosmétiquement acceptable des compositions selon l'invention est de préférence constitué d'eau, d'un ou plusieurs solvants organiques cosmétiquement acceptables ou bien d'un mélange d'eau et d'un ou plusieurs solvants organiques cosmétiquement acceptables.

Parmi ces solvants organiques, on utilise plus particulièrement les alcools inférieurs en C₁-C₄ tels que l'éthanol notamment dans les produits capillaires. Pour les vernis à ongles ou les bases de soin des ongles, on utilise plus particulièrement les mélanges d'acétate de butyle et d'acétate d'éthyle.

Le support peut être également constitué de corps gras tels que les huiles minérales, végétales animales ou de synthèse, les cires animales, fossiles, végétales, minérales ou de synthèse.

Les polymères filmogènes selon l'invention sont dissous ou en dispersion dans le support des compositions de l'invention.

Les agents plastifiants conformes à l'invention sont dissous ou en dispersion dans le support des compositions de l'invention.

Les compositions peuvent en outre, et bien entendu, contenir divers adjuvants destinés à la rendre acceptable dans une application cosmétique particulière.

Les compositions selon l'invention peuvent contenir des additifs cosmétiques conventionnels choisis parmi les corps gras tels que les huiles minérales, végétales animales ou de synthèse, les cires animales, fossiles, végétales, minérales ou de synthèse, des solvants organiques, des agents épaississants, des adoucissants, des agents anti-mousse, des agents hydratants, des humectants, des agents traitants (agents anti-chute, anti-pelliculaire, ...), des antiperspirants, des agents alcanisants, des filtres solaires UV-A ou UV-B ou à bande large, des colorants, des pigments, des parfums, des conservateurs, des polymères organiques anioniques, non-ioniques ou amphotères compatibles avec les copolymères greffés de l'invention et des agents propulseurs lorsque les compositions se présentent sous forme aérosol.

Bien entendu, l'homme de l'art veillera à choisir le ou les éventuels composés complémentaires mentionnés ci-avant, de manière telle que les propriétés avantageuses attachées intrinsèquement aux compositions selon l'invention ne soient pas, ou substantiellement pas, altérées par la ou les adjonctions envisagées.

L'invention a également pour objet un procédé de traitement cosmétique des matières kératiniques telles que la peau, les cheveux, le cuir chevelu, les cils, les sourcils, les ongles, les lèvres, caractérisé en ce qu'il consiste à appliquer sur ces dernières une composition telle que définie ci-dessus.

Les exemples qui suivent servent à illustrer l'invention sans toutefois présenter un caractère limitatif.

### EXEMPLES DE PREPARATION

### EXEMPLE DE PREPARATION 1 :

### Synthèse d'un polyester polysébaçate de néopentylglycol de poids moléculaire moyen de 600 et à extrémités α, ω hydroxyles.

### Composition des réactifs de départ :

| | |
|---|---|
| - Diméthyl sébaçate | 276 g (1,2 M) |
| - 2,2-diméthyl-1,3-propanediol (appelé aussi néopentylglycol) | 274,6 g (2,64 M) |
| - Acétate de zinc dihydraté 0,3% en poids/charge | 1,6 g |
| - 1,2-dichloroéthane pur | 2 l |
| - Eau permutée | 2 l |

### MODE OPERATOIRE

On utilise un réacteur de 500 ml cylindrique muni d'une arrivée d'azote, d'un thermomètre et d'un montage de distillation. Le réacteur est chauffé par un bain en alliage de Wood.
On introduit dans le réacteur le 2,2-diméthyl-1,3-propanediol (point de fusion 126-128°C) et le diméthylsébaçate (point de fusion 25-28°C) préalablement fondu. On chauffe le mélange de la température ambiante à 150°C en 1 heure. Le milieu réactionnel devient limpide vers 100°C. Dès que l'on atteint 150°C dans la réaction, on introduit alors l'acétate de zinc. On laisse se développer la synthèse pendant 3 heures à 150°C tout en recueillant le méthanol formé. Ensuite, on monte la température à 200°C en 45 minutes et on maintient alors 3 heures à 200°C. La distillation du méthanol continue durant toute la condensation.

A la fin des 3 heures à 200°C, on laisse revenir à la température ambiante en réduisant l'agitation. Dès que la température intérieure arrive à 50°C, on ajoute 300 ml de 1,2-dichloroéthane.

On récupère la solution de synthèse que l'on dilue par 1,7 l de1,2-dichloroéthane. L'excès de 2,2-diméthyl-1,3-propanediol est alors extrait deux fois par 1 litre d'eau permutée. Durant ces deux extractions, il est possible qu'il se forme une émulsion interfaciale. Cette émulsion est facilement éliminable par chauffage de l'eau utilisée en cours de l'extraction. On récupère la phase organique que l'on sèche sur sulfate de sodium anhydre. On filtre et on évapore le1,2-dichloroéthane jusqu'à la récupération du produit sec.

Ce polymère se présente sous forme de liquide visqueux à température ambiante.

### CARACTERISATION :

| | |
|---|---|
| Indice d'hydroxyle : | 190-195 |
| Poids moléculaire mesuré : | 600 |
| en sommet de pic par chromatographie par exclusion stérique | |

### EXEMPLE DE PREPARATION 2 :

### Synthèse d'un copolymère sébaçate/téréphtalate de néopentylglycol de poids moléculaire moyen de 600-700 et à extrémités α,ω-hydroxyles.

### Composition des réactifs de départ :

| | |
|---|---|
| - Diméthyl téréphtalate | 116,4 g (0,6 M) |
| - Diméthyl sébaçate | 138 g (0,6 M) |
| - 2,2-diméthyl-1,3-propanediol (appelé aussi néopentylglycol) | 274,6 g (2,64 M) |
| - Acétate de zinc dihydraté 0,3% en poids/réactif | 1,6 g |
| - 1,2-dichloroéthane pur | 2 l |
| - Eau permutée | 2 l |

### MODE OPERATOIRE

On utilise un réacteur de 500 ml cylindrique muni d'une arrivée d'azote, d'un thermomètre et d'un montage de distillation. Le réacteur est chauffé par un bain en alliage de Wood.
On introduit dans le réacteur le 2,2-diméthyl-1,3-propanediol (point de fusion 126-128°C) et le diméthylsébaçate (point de fusion 25-28°C) préalablement fondu. On chauffe le mélange de la température ambiante à 150°C en 1 heure. Dès que le milieu réactionnel devient limpide vers 100°C, on ajoute le diméthyl téréphtalate. Dès que l'on atteint 150°C dans la réaction, on introduit alors l'acétate de zinc. On laisse se développer la synthèse pendant 3 heures à 150°C tout en recueillant le méthanol formé. Ensuite, on monte la température à 200°C en 45 minutes et on maintient alors 3 heures à 200°C. La distillation du méthanol continue durant toute la condensation.
A la fin des 3 heures à 200°C, on laisse revenir à la température ambiante en réduisant l'agitation. Dès que la température intérieure arrive à 50°C, on ajoute 300 ml de 1,2-dichloroéthane.
On récupère la solution de synthèse que l'on dilue par 1,7 l de 1,2-dichloroéthane. L'excès de 2,2-diméthyl-1,3-propanediol est alors extrait deux fois par 1 litre d'eau permutée. Durant ces deux extractions, il est possible qu'il se forme une émulsion interfaciale. Cette émulsion est facilement éliminable par chauffage de l'eau utilisée en cours de l'extraction. On récupère la phase organique que l'on sèche sur sulfate de sodium anhydre. On filtre et on évapore le 1,2-dichloroétane jusqu'à la récupération du produit sec.

Le polymère se présente sous forme de pâte à température ambiante et devient liquide à 50°C.

### CARACTERISATION :

| | |
|---|---|
| Indice d'hydroxyle : | 189-192 |
| Poids moléculaire mesuré : | 600 |
| en sommet de pic par chromatographie par exclusion stérique | |

### EXEMPLES D'APPLICATION

### EXEMPLE 3: Laque aérosol

### Composition de la laque :

| | |
|---|---|
| - Copolymère acétate de vinyle/tertio-butyl benzoate de vinyle/acide crotonique(65/25/10 % en poids) de poids moléculaire 100.000 décrit dans le brevet FR 2 439 798 (filmogène) | 8,6 g |
| - Amino-2-méthyl-2-propanol (neutralisant) | 0,86 g |
| - Oligomère polyester de l'exemple 1 (plastifiant) | 0,43 g |
| - Ethanol absolu qs | 100 g |

### Pressurisation :

| | |
|---|---|
| - Composition de la laque | 37 g |
| - Diméthyléther | 43 g |
| - Pentane | 20 g |

Cette formulation, vaporisée sur les cheveux, en comparaison avec une formulation contenant un plastifiant classique du type polyéther de glycol dans la même quantité, conduit pour un niveau de fixation équivalent à un démêlage plus facile, une douceur supérieure et un toucher du cheveu nettement plus agréable, plus lisse, plus léger et sans effet de charge.

### EXEMPLE 4: Laque aérosol

### Composition de la laque :

| | |
|---|---|
| - Copolymère acide acrylique/acrylate d'éthyle/N-tertiobutylacrylamide vendu sous le nom ULTRAHOLD STRONG par BASF | 12 g |
| - Amino-2-méthyl-2-propanol (neutralisant) | 1,5 g |
| - Oligomère polyester de l'exemple 1 (plastifiant) | 0,6 g |
| - Eau | 33,5 g |
| - Ethanol absolu qs | 100 g |

### Pressurisation :

| | |
|---|---|
| - Composition de la laque | 45 % |
| - Diméthyléther | 55 % |

Cette formulation, vaporisée sur les cheveux, en comparaison avec une formulation contenant un plastifiant classique du type polyéther de glycol dans la même quantité, conduit pour un niveau de fixation équivalent à un démêlage plus facile, une douceur supérieure et un toucher du cheveu nettement plus agréable, plus lisse, plus léger et sans effet de charge.

### EXEMPLE 5 : Laque aérosol

### Composition de la laque :

| | |
|---|---|
| - Copolymère acétate de vinyle/tertio-butyl benzoate de vinyle/acide crotonique(65/25/10 % en poids) de poids moléculaire décrit dans le brevet FR 2 439 798 (filmogène) | 5,3 g |
| - Amino-2-méthyl-2-propanol (neutralisant) | 0,54 g |
| - Oligomère polyester de l'exemple 2 (plastifiant) | 0,16 g |
| - Ethanol absolu | 30,8 g |
| - Eau qs | 100 g |

### Pressurisation :

| | |
|---|---|
| - Composition de la laque | 65 % |
| - Diméthyléther | 35 % |

Cette formulation conduit à de bonnes propriétés de fixation des cheveux avec un toucher de cheveu lisse et propre et moins sec que les formulations classiques à base de plastifiants courants.

### EXEMPLE 6: Spray en flacon pompe

| | |
|---|---|
| - Terpolymère acide acrylique/acrylate d'éthyle/acrylate de tertiobutyle vendu sous le nom LUVIMER 100P par la société BASF (filmogène) | 7,0 g |
| - Amino-2-méthyl-2-propanol (neutralisant) qs | neutralisation à 100% |
| - Oligomère polyester de l'exemple 2 (plastifiant) | 0,35 g |
| - Ethanol absolu qsp | 100 g |

Cette formulation conduit à de bonnes propriétés de fixation des cheveux avec un toucher de cheveu lisse et propre et moins sec que les formulations classiques à base de plastifiants courants.

### EXEMPLE 7 : Laque aérosol

### Composition de la laque :

| | |
|---|---|
| - Copolymère N-octylacrylamide/méthacrylate de méthyle/méthacrylate d'hydroxypropyle/acide acrylique/méthacrylate de tert-butyl aminoéthyle vendu sous le nom AMPHOMER LV 71 par NATIONAL STARCH (filmogène) | 15 g |
| - Amino-2-méthyl-2-propanol (neutralisant) qs | neutralisation à 100% |
| - Oligomère polyester de l'exemple 1 (plastifiant) | 0,7 g |
| - Eau | 33,5 g |
| - Ethanol absolu qs | 100 g |

### Pressurisation :

| | |
|---|---|
| - Composition de la laque | 45 % |
| - Diméthyléther | 55 % |

Cette formulation conduit à de bonnes propriétés de fixation des cheveux avec un toucher de cheveu lisse et propre et moins sec que les formulations classiques à base de plastifiants courants.

### EXEMPLE 8 : Vernis à ongle

| | |
|---|---|
| - Copolymère greffé chloré décrit dans l'exemple 1 du brevet FR 9213600 (filmogène) | 23 % |
| - Heptane | 10 % |
| - Acétate d'éthyle | 20% |
| - Acétate de butyle | 34,9% |
| - Oligomère polyester de l'exemple 1 (plastifiant) | 10 % |
| - Pigments | 0,8% |
| - Agent thixotrope | 1,2% |
| - Acide citrique | 0,1% |

Le vernis obtenu est brillant et a une bonne tenue.

### EXEMPLE 9 : Vernis à ongle

| | |
|---|---|
| - Copolymère greffé chloré décrit dans l'exemple 1 du brevet FR 92 13600 (filmogène) | 23 % |
| - Heptane | 10 % |
| - Acétate d'éthyle | 20% |
| - Acétate de butyle | 34,9% |
| - Oligomère polyester de l'exemple 2 (plastifiant) | 10 % |
| - Pigments | 0,8% |
| - Agent thixotrope | 1,2% |
| - Acide citrique | 0,1% |

Le vernis obtenu est brillant et a une bonne tenue.

## Revendications

1. Utilisation, comme agent plastifiant d'un polymère filmogène, d'au moins un oligomère choisi parmi les homopolymères ou copolymères de monomère(s) à insaturation éthylénique et les polycondensats, ayant un poids moléculaire moyen, mesuré en sommet de pic par chromatographie par exclusion stérique, allant de 400 à 10000 et ayant une température de transition vitreuse allant de -80 à 10°C, dans une composition cosmétique ou dermatologique contenant au moins ce polymère filmogène ; ladite composition ne contenant pas de polymère filmogène du type nitrocellulose.

2. Utilisation selon la revendication 1, caractérisée par le fait que le poids moléculaire moyen de l'oligomère plastifiant, mesuré en sommet de pic par chromatographie par exclusion stérique, varie de 500 à 5000.

3. Utilisation selon la revendication 1 ou 2, caractérisée par le fait que la température de transition vitreuse de l'oligomère plastifiant varie de -70 à 5°C.

4. Utilisation selon l'une quelconque des revendications 1 à 3, caractérisée par le fait que l'oligomère plastifiant est choisi parmi les homopolymères ou copolymères acryliques, méthacryliques, allyliques, méthallyliques ou vinyliques, anioniques, cationiques, amphotères ou non-ioniques.

5. Utilisation selon l'une quelconque des revendications 1 à 4, caractérisée par le fait que l'oligomère plastifiant est choisi parmi les polymères d'au moins un monomère choisi dans le groupe constitué par :
- les esters ou les amides acryliques ou méthacryliques obtenus à partir d'alcools aliphatiques, linéaires, ramifiés ou cycliques et/ou d'alcools aromatiques, de préférence en C₁-C₃₀ ;
- les esters ou amides vinyliques, allyliques ou méthallyliques obtenus à partir d'alcools aliphatiques, linéaires, ramifiés ou cycliques et/ou d'alcools aromatiques, de préférence en C₁-C₃₀ ;
- les oléfines ;
- les monomères acryliques ou vinyliques halogénés ou perhalogénés et plus particulièrement fluorés ou perfluorés ;
- leurs mélanges.

6. Utilisation selon l'une quelconque des revendications 1 à 4, caractérisée par le fait que l'oligomère plastifiant est choisi parmi les polymères d'au moins un monomère choisi dans le groupe constitué par :
- les monomères comportant au moins une fonction acide, sous forme libre ou bien sous forme partiellement ou totalement neutralisée tels que les mono acides carboxyliques comme les acides acrylique, méthacrylique, crotonique ; les diacides carboxyliques ou les anhydrides d'acide ainsi que leurs monoesters ou monoamides comme l'anhydride maléique sous forme de diacide, de monoester ou monoamide, l'acide itaconique ;
- les monomères comportant au moins une fonction acide sulfonique, sous forme libre ou bien sous forme partiellement ou totalement neutralisée tels que l'acide vinyl-ou styrène sulfonique, l'acide acrylamido-2 méthylpropane-2 sulfonique ;
- les monomères comportant au moins une fonction acide phosphorique ou phosphonique, sous forme libre ou bien sous forme partiellement ou totalement neutralisée.

7. Utilisation selon l'une quelconque des revendications 1 à 4, caractérisée par le fait que l'oligomère plastifiant est choisi parmi les polymères d'au moins un monomère choisi dans le groupe constitué par :
- les monomères comportant une fonction amine sous forme libre ou bien partiellement ou totalement neutralisée ou bien partiellement ou totalement quaternisée.

8. Utilisation selon l'une quelconque des revendications 1 à 4, caractérisée par le fait que l'oligomère plastifiant est choisi parmi les polymères d'au moins un monomère choisi dans le groupe constitué par :
- les carboxybétaïnes ou les sulfobétaïnes obtenues par quaternisation partielle ou totale de monomères à insaturation éthylénique comportant une fonction amine par des sels de sodium d'acide carboxylique à halogénure mobile ou par des sultones cycliques.

9. Utilisation selon l'une quelconque des revendications 1 à 4, caractérisée par le fait que l'oligomère plastifiant est choisi parmi les polymères d'au moins un monomère choisi dans le groupe constitué par :
- les (méth)acrylate d'hydroxyalkyle en C₁-C₃₀ ;
- les (méth)acrylamides, les dialkyl(C₁-C₃₀) (méth)acrylamides ;
- la N-vinylpyrrolidone, la vinylcaprolactam ;
- les (méth)acrylates d'éthylèneglycol, de diéthylèneglycol, de polyéthylèneglycol à
extrémité hydroxyle ou éther.

10. Utilisation selon l'une quelconque des revendications 1 à 3, caractérisée par le fait que l'oligomère plastifiant est choisi parmi les polyesters, les polyester/amides, les polyamides, les polyuréthanes et les polyurées ou leurs mélanges ; ledit oligomère étant anionique, cationique, amphotère ou non-ionique.

11. Utilisation selon la revendication 10, caractérisée par le fait que les polyesters sont choisis dans le groupe constitué par :
- (i) les composés de formule (1) suivante :
HOOC-(CH₂)ₘ-COOH (1)
où m est un nombre entier allant de 2 à 20 ;
- (ii) les composés de formule (2) suivante : 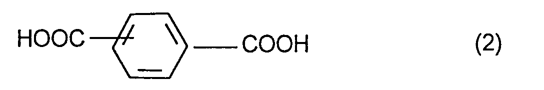 le groupe acide mobile étant de préférence en position ortho, para ou méta et le cycle benzénique pouvant comporter d'autres substituants différents des groupes sulfonates ;
- (iii) leurs mélanges , avec un diol choisi dans le groupe constitué par :
- (a) les composés de formule (3) suivante :
HO-(CH₂)ₓ-OH (3)
où x est un nombre entier allant de 2 à 20 ;
- (b) les composés de formule (4) suivante : 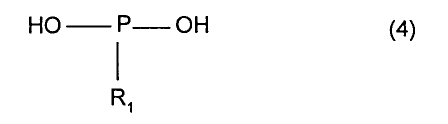 où P est un radical hydrocarboné trivalent aliphatique, linéaire ou ramifié, cycloaliphatique ou aromatique, de préférence un groupe -(CH₂)ₚ-CH- avec p ≥ 1 et plus particulièrement le groupe -CH₂-CH- et R₁ désigne une chaîne latérale hydrocarbonée en C₂-C₃₀ ;
- (c) les composés de formule (5) suivante : 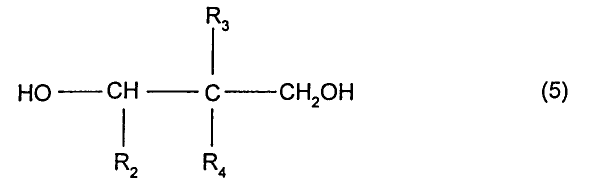 où R₂ désigne un atome d'hydrogène ou un radical alkyle linéaire ou ramifié en C₁-C₃ ; R₃ désigne un atome d'hydrogène ou un radical alkyle linéaire ou ramifié en C₁-C₄ et R₄ désigne un radical alkyle linéaire ou ramifié en C₁-C₄ ;
- (d) le composé de formule (6) suivante : 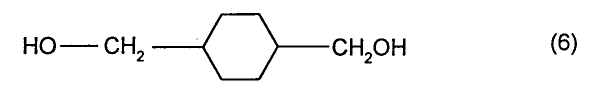
- (e) le composé de formule (7) suivante : 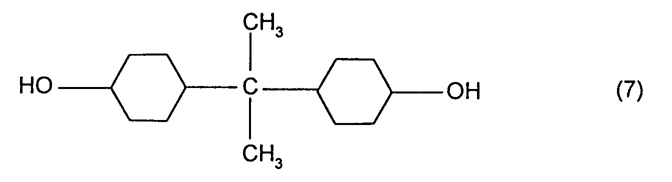
- (f) leurs mélanges.

12. Composition cosmétique ou dermatologique contenant dans un support cosmétiquement ou dermatologiquement acceptable au moins un polymère filmogène, dissout ou dispersé dans ledit support et au moins un oligomère tel que défini dans l'une quelconque des revendications 1 à 11, dissout ou dispersé dans ledit support, comme agent plastifiant dudit polymère filmogène ; ladite composition ne contenant pas de nitrocellulose.

13. Composition selon la revendication 12, caractérisée par le fait que l'oligomère plastifiant est utilisé dans des concentrations en matières sèches allant de 0,1 à 80 % en poids, plus préférentiellement de 5 à 40% et plus particulièrement de 10 à 30% en poids par rapport au poids total de la composition.

14. Composition selon la revendication 12 ou 13, caractérisée par le fait que le polymère filmogène est choisi dans le groupe constitué par :
- les copolymères acétate de vinyle/acide crotonique/tertio-butyl benzoate de vinyle ;
- les copolymères acide acrylique/acrylate d'éthyle /N-tertiobutylacrylamide ;
- les copolymères N-octylacrylamide/méthacrylate de méthyle/méthacrylate d'hydroxypropyle/acide acrylique/méthacrylate de tert-butyl aminoéthyle ;.
- les terpolymères acide acrylique/acrylate d'éthyle/acrylate de tertiobutyle.

15. Composition selon l'une quelconque des revendications 12 à 14, caractérisée par le fait que la quantité de polymère filmogène dans les compositions cosmétiques ou dermatologiques de l'invention varie de 0,1 à 50%, et de préférence entre 1 et 30% en poids par rapport au poids total de la composition.

16. Composition selon l'une quelconque des revendications 12 à 15, caractérisée par le fait que le support cosmétiquement ou dermatologiquement acceptable constitué d'eau, d'un ou plusieurs solvants organiques cosmétiquement acceptables ou bien d'un mélange d'eau et d'un ou plusieurs solvants organiques cosmétiquement acceptables ou bien de corps gras choisi parmi les huiles minérales, végétales, animales ou de synthèse, les cires animales, fossiles, végétales, minérales ou de synthèse.

17. Composition selon la revendication 16, où les solvants organiques sont des alcools inférieurs en C₁-C₄ ou des mélanges d'acétate de butyle et d'acétate d'éthyle.

18. Composition selon l'une quelconque des revendications 12 à 17, caractérisée par le fait qu'elle contient des additifs cosmétiques conventionnels choisis parmi les corps gras tels que les huiles minérales, végétales animales ou de synthèse, les cires animales, fossiles, végétales, minérales ou de synthèse, des solvants organiques, des agents épaississants, des adoucissants, des agents anti-mousse, des agents hydratants, des humectants, des agents traitants, des antiperspirants, des agents alcanisants, des agents acidifiants, des filtres solaires UV-A ou UV-B ou à bande large, des colorants, des pigments, des parfums, des conservateurs, des polymères organiques anioniques, non-ioniques ou amphotères et les agents propulseurs

19. Composition selon l'une quelconque des revendications 12 à 18, caractérisée en ce qu'il s'agit d'une composition capillaire.

20. Composition selon l'une quelconque des revendications 12 à 18, caractérisée en ce qu'il s'agit d'une composition de maquillage.

21. Composition selon l'une quelconque des revendications 12 à 18, caractérisée en ce qu'il s'agit d'une composition pour le soin de la peau.

22. Procédé de traitement cosmétique des matières kératiniques, caractérisé en ce qu'il consiste à appliquer sur ces dernières une composition cosmétique telle que définie à l'une quelconque des revendications 12 à 21.

## Claims

1. Use as plasticizer of a film-forming polymer of at least one oligomer chosen from homopolymers or copolymers of monomer(s) containing ethylenic unsaturation and polycondensates, having an average molecular weight, measured at the peak height by steric exclusion chromatography, ranging from 400 to 10,000 and having a glass transition temperature ranging from -80 to 10°C, in a cosmetic or dermatological composition containing at least this film-forming polymer, the said composition containing no film-forming polymer of the nitrocellulose type.

2. Use according to Claim 1, characterized in that the average molecular weight of the plasticizing oligomer, measured at the peak height by steric exclusion chromatography, ranges from 500 to 5000.

3. Use according to Claim 1 or 2, characterized in that the glass transition temperature of the plasticizing oligomer ranges from -70 to 5°C.

4. Use according to any one of Claims 1 to 3, characterized in that the plasticizing oligomer is chosen from an anionic, cationic, amphoteric or nonionic acrylic, methacrylic, allylic, methallylic or vinyl homopolymers or copolymers.

5. Use according to any one of Claims 1 to 4, characterized in that the plasticizing oligomer is chosen from polymers of at least one monomer chosen from the group consisting of:
- acrylic or methacrylic esters or amides obtained from linear, branched or cyclic aliphatic alcohols and/or from aromatic alcohols, which are preferably C₁-C₃₀,
- vinyl, allylic or methallylic esters or amides obtained from linear, branched or cyclic aliphatic alcohols and/or from aromatic alcohols, which are preferably C₁-C₃₀,
- olefins;
- halo or perhalo and more particularly fluoro or perfluoro acrylic or vinyl monomers;
- mixtures thereof.

6. Use according to any one of Claims 1 to 4, characterized in that the plasticizing oligomer is chosen from polymers of at least one monomer chosen from the group consisting of:
- monomers containing at least one acid function, in free form or in partially or totally neutralized form, such as monocarboxylic acids, for instance acrylic acid, methacrylic acid or crotonic acid; dicarboxylic acids or acid anhydrides as well as the monoesters or monoamides thereof, for instance maleic anhydride in diacid, monoester or monoamide form, and itaconic acid;
- monomers containing at least one sulphonic acid function, in free form or in partially or totally neutralized form, such as vinylsulphonic or styrenesulphonic acid or 2-acrylamido-2-methylpropanesulphonic acid;
- monomers containing at least one phosphoric or phosphonic acid function, in free form or in partially or totally neutralized form.

7. Use according to any one of Claims 1 to 4, characterized in that the plasticizing oligomer is chosen from polymers of at least one monomer chosen from the group consisting of:
- monomers containing an amine function in free form or partially or totally neutralized or alternatively partially or totally quaternized.

8. Use according to any one of Claims 1 to 4, characterized in that the plasticizing oligomer is chosen from polymers of at least one monomer chosen from the group consisting of:
- carboxybetaines or sulphobetaines obtained by partial or total quaternization of monomers containing ethylenic unsaturation and containing an amine function, with sodium salts of a carboxylic acid containing a labile halide, or with cyclic sultones.

9. Use according to any one of Claims 1 to 4, characterized in that the plasticizing oligomer is chosen from polymers of at least one monomer chosen from the group consisting of:
- hydroxy(C₁-C₃₀)alkyl (meth)acrylates;
- (meth)acrylamides, di(C₁-C₃₀)alkyl (meth)acrylamides;
- N-vinylpyrrolidone, vinylcaprolactam;
- (meth)acrylates of ethylene glycol, of diethylene glycol or of polyethylene glycol containing a hydroxyl or ether end.

10. Use according to any one of Claims 1 to 3, characterized in that the plasticizing oligomer is chosen from polyesters, polyester/amides, polyamides, polyurethanes and polyureas or mixtures thereof, the said oligomer being anionic, cationic, amphoteric or nonionic.

11. Use according to Claim 10, characterized in that the polyesters are chosen from the group consisting of:
- (i) the compounds of formula (1) below:
HOOC-(CH₂)ₘ-COOH (1)
where m is an integer ranging from 2 to 20;
- (ii) the compounds of formula (2) below: 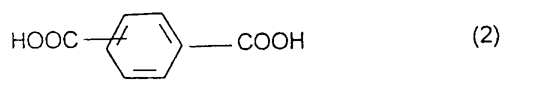 the labile acid group preferably being in the ortho, para or meta position and it being possible for the benzene ring to contain substituents other than sulphonate groups;
- (iii) mixtures thereof, with a diol chosen from the group consisting of:
- (a) the compounds of formula (3) below:
HO-(CH₂)ₓ-OH (3)
where x is an integer ranging from 2 to 20;
- (b) the compounds of formula (4) below: 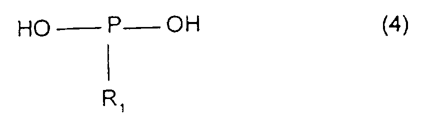 where P is an aromatic, cycloaliphatic or linear or branched aliphatic trivalent hydrocarbon radical, preferably a group -(CH₂)ₚ-CH- with p ≥ 1 and more particularly the -CH₂-CH- group and R₁ denotes a C₂-C₃₀ hydrocarbon side chain;
- (c) the compounds of formula (5) below: 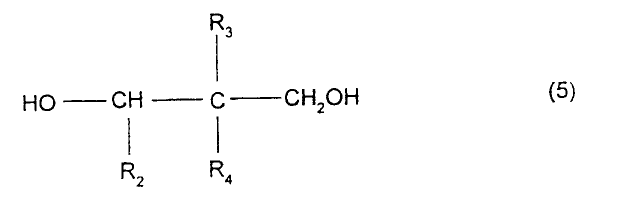 where R₂ denotes a hydrogen atom or a linear or branched C₁-C₃ alkyl radical; R₃ denotes a hydrogen atom or a linear or branched C₁-C₄ alkyl radical and R₄ denotes a linear or branched C₁-C₄ alkyl radical;
- (d) the compound of formula (6) below: 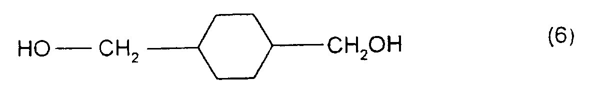
- (e) the compound of formula (7) below: 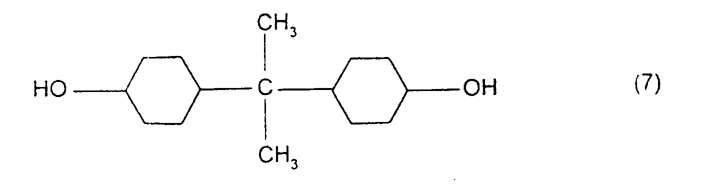
- (f) mixtures thereof.

12. Cosmetic or dermatological composition containing, in a cosmetically or dermatologically acceptable vehicle, at least one film-forming polymer which is dissolved or dispersed in the said vehicle and at least one oligomer as defined in any one of Claims 1 to 11, which is dissolved or dispersed in the said vehicle, as a plasticizer for the said film-forming polymer, the said composition containing no nitrocellulose.

13. Composition according to Claim 12, characterized in that the plasticizing oligomer is used in solids concentrations ranging from 0.1 to 80% by weight, more preferably from 5 to 40% and more particularly from 10 to 30% by weight, relative to the total weight of the composition.

14. Composition according to Claim 12 or 13, characterized in that the film-forming polymer is chosen from the group consisting of:
- the vinyl acetate/crotonic acid/vinyl tert-butylbenzoate copolymers;
- acrylic acid/ethyl acrylate/N-tert-butylacrylamide copolymers;
- N-octylacrylamide/methyl methacrylate/hydroxypropyl methacrylate/acrylic acid/tert-butylaminoethyl methacrylate copolymers;
- acrylic acid/ethyl acrylate/tert-butyl acrylate terpolymers.

15. Composition according to any one of Claims 12 to 14, characterized in that the amount of film-forming polymer in the cosmetic or dermatological compositions of the invention ranges from 0.1 to 50%, and is preferably between 1 and 30%, by weight relative to the total weight of the composition.

16. Composition according to any one of Claims 12 to 15, characterized in that the cosmetically or dermatologically acceptable vehicle consists of water or of one or more cosmetically acceptable organic solvents or alternatively of a mixture of water and one or more cosmetically acceptable organic solvents or alternatively of fatty substances chosen from mineral oils, plant oils, animal oils or synthetic oils and animal waxes, fossil waxes, plant waxes, mineral waxes or synthetic waxes.

17. Composition according to Claim 16, in which the organic solvents are C₁-C₄ lower alcohols or mixtures of butyl acetate and ethyl acetate.

18. Composition according to any one of Claims 12 to 17, characterized in that it contains conventional cosmetic additives chosen from fatty substances such as mineral oils, plant oils, animal oils or synthetic oils, animal waxes, fossil waxes, plant waxes, mineral waxes or synthetic waxes, organic solvents, thickeners, softeners, antifoaming agents, hydrating agents, wetting agents, treating agents, antiperspirants, basifying agents, acidifying agents, UV-A or UV-B or broad-band sunscreens, dyes, pigments, fragrances, preserving agents, anionic, nonionic or amphoteric organic polymers and propellants.

19. Composition according to any one of Claims 12 to 18, characterized in that it is a hair composition.

20. Composition according to any one of Claims 12 to 18, characterized in that it is a make-up composition.

21. Composition according to any one of Claims 12 to 18, characterized in that it is a skincare composition.

22. Process for the cosmetic treatment of keratin substances, characterized in that it consists in applying a cosmetic composition as defined in any one of Claims 12 to 21 to these keratin substances.

## Patentansprüche

1. Verwendung mindestens eines Oligomers, das unter den Homopolymeren oder Copolymeren eines ethylenisch ungesättigten Monomers oder ethylenisch ungesättigter Monomere und Polykondensaten ausgewählt ist, die ein durch Ausschlußchromatographie bezogen auf die Peakwerte bestimmtes mittleres Molekulargewicht im Bereich von 400 bis 10000 und eine Glasübergangstemperatur im Bereich von -80 bis 10 °C aufweisen, als Weichmacher für ein filmbildendes Polymer in einer kosmetischen oder dermatologischen Zusammensetzung, die zumindest dieses filmbildende Polymer enthält, wobei die Zusammensetzung kein filmbildendes Polymer vom Nitrocellulosetyp enthält.

2. Verwendung nach Anspruch 1, dadurch gekennzeichnet, daß das durch Ausschlußchromatographie bezogen auf die Peakwerte bestimmte mittlere Molekulargewicht des weichmachenden Oligomers im Bereich von 500 bis 5000 liegt.

3. Verwendung nach Anspruch 1 oder 2, dadurch gekennzeichnet, daß die Glasübergangstemperatur des weichmachenden Oligomers im Bereich von -70 bis 5 °C liegt.

4. Verwendung nach einem der Ansprüche 1 bis 3, dadurch gekennzeichnet, daß das weichmachende Polymer unter anionischen, kationischen, amphoteren oder nichtionischen Acryl-, Methacryl-, Allyl-, Methallyl- oder Vinylhomopolymeren oder -copolymeren ausgewählt ist.

5. Verwendung nach einem der Ansprüche 1 bis 4, dadurch gekennzeichnet, daß das weichmachende Oligomer ausgewählt ist unter den Polymeren mindestens eines Monomers, das ausgewählt ist unter:
- Acrylestern, Methacrylestern, Acrylamiden oder Methacrylamiden, die ausgehend von geradkettigen, verzweigten oder cyclischen aliphatischen Alkoholen und/oder aromatischen Alkoholen hergestellt werden, wobei sie vorzugsweise 1 bis 30 Kohlenstoffatome aufweisen;
- Vinylestern, Allylestern, Methallylestern, Vinylamiden, Allylamiden oder Methallylamiden, die ausgehend von geradkettigen, verzweigten oder cyclischen aliphatischen Alkoholen und/oder aromatischen Alkoholen erhalten werden, wobei sie vorzugsweise 1 bis 30 Kohlenstoffatome aufweisen;
- Olefinen;
- halogenierten oder perhalogenierten und insbesondere fluorierten oder perfluorierten Acrylmonomeren oder Vinylmonomeren und
- deren Gemischen.

6. Verwendung nach einem der Ansprüche 1 bis 4, dadurch gekennzeichnet, daß das weichmachende Oligomer ausgewählt ist unter den Polymeren mindestens eines Monomers, das ausgewählt ist unter:
- Monomeren, die mindestens eine Säuregruppe aufweisen, in freier Form oder auch in teilweise oder vollständig neutralisierter Form, wie Monocarbonsäuren, wie beispielsweise Acrylsäure, Methacrylsäure oder Crotonsäure, Dicarbonsäuren oder Säureanhydride sowie ihre Monoester oder Monoamide, wie beispielsweise Maleinsäureanhydrid in Disäureform, Monoesterform oder Monoamidform oder Itaconsäure;
- Monomeren, die mindestens eine Sulfonsäuregruppe aufweisen, in freier Form oder auch in teilweise oder vollständig neutralisierter Form, wie Vinylsulfonsäure, Styrolsulfonsäure oder 2-Acrylamido-2-methylpropansulfonsäure und
- Monomeren, die mindestens eine Phosphorsäuregruppe oder eine Phosphonsäuregruppe aufweisen, in freier Form oder auch in teilweise oder vollständig neutralisierter Form.

7. Verwendung nach einem der Ansprüche 1 bis 4, dadurch gekennzeichnet, daß das weichmachende Oligomer ausgewählt ist unter den Polymeren mindestens eines Monomers, das ausgewählt ist unter:
- Monomeren, die eine Aminogruppe aufweisen, in freier oder auch teilweise oder vollständig neutralisierter Form oder auch teilweise oder vollständig quaternisierter Form.

8. Verwendung nach einem der Ansprüche 1 bis 4, dadurch gekennzeichnet, daß das weichmachende Oligomer ausgewählt ist unter den Polymeren mindestens eines Monomers, das ausgewählt ist unter:
- Carboxybetainen oder Sulfobetainen, die durch teilweise oder vollständige Quaternisierung von ethylenisch ungesättigten Monomeren, die eine Aminogruppe aufweisen, durch Natriumsalze einer Carbonsäure mit mobilem Halogenid oder durch cyclische Sultone erhalten werden.

9. Verwendung nach einem der Ansprüche 1 bis 4, dadurch gekennzeichnet, daß das weichmachende Oligomer ausgewählt ist unter den Polymeren mindestens eines Monomers, das ausgewählt ist unter:
- Hydroxyalkyl(meth)acrylaten mit 1 bis 30 Kohlenstoffatomen;
- (Meth)acrylamiden und (C₁₋₃₀)-Dialkyl(meth)acrylamiden;
- N-Vinylpyrrolidon und Vinylcaprolactam und
- (Meth)acrylaten von Ethylenglykol, Diethylenglykol und Polyethylenglykol mit Hydroxyendgruppe oder Etherendgruppe.

10. Verwendung nach einem der Ansprüche 1 bis 3, dadurch gekennzeichnet, daß das weichmachende Oligomer unter Polyestern, Polyester/amiden, Polyamiden, Polyurethanen und Polyharnstoffen oder deren Gemischen ausgewählt ist, wobei das Oligomer anionisch, kationisch, amphoter oder nichtionisch ist.

11. Verwendung nach Anspruch 10, dadurch gekennzeichnet, daß die Polyester ausgewählt sind unter:
- (i) den Verbindungen der folgenden Formel (1):
HOOC-(CH₂)ₘ-COOH (1),
worin m eine ganze Zahl im Bereich von 2 bis 20 bedeutet;
- (ii) den Verbindungen der folgenden Formel (2): 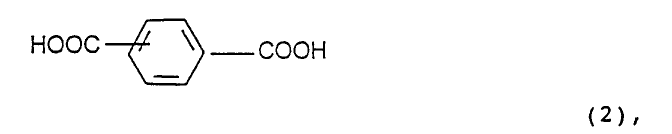 wobei sich die Säuregruppe, deren Stellung nicht definiert ist, vorzugsweise in ortho-, para- oder meta-Stellung befindet und wobei der Benzolring weitere Substituenten aufweisen kann, die von Sulfonatgruppen verschieden sind, und
- (iii) den Gemischen dieser Verbindungen, mit einem Diol, das ausgewählt ist unter:
- (a) den Verbindungen der folgenden Formel (3):
HO-(CH₂)ₓ-OH (3),
worin x eine ganze Zahl im Bereich von 2 bis 20 bedeutet,
- (b) den Verbindungen der folgenden Formel (4): 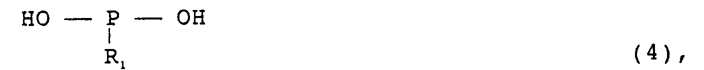 worin P eine dreiwertige geradkettige oder verzweigte aliphatische, cycloaliphatische oder aromatische Kohlenwasserstoffgruppe, vorzugsweise eine Gruppe -(CH₂)ₚ-CH- mit p ≥ 1 und insbesondere die Gruppe -CH₂-CH- und R₁ eine Kohlenwasserstoffseitenkette mit 2 bis 30 Kohlenstoffatomen bedeuten;
- (c) den Verbindungen der folgenden Formel (5): 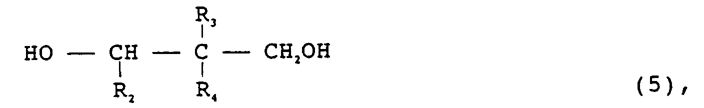 worin R₂ ein Wasserstoffatom oder eine geradkettige oder verzweigte C₁₋₃-Alkylgruppe, R₃ ein Wasserstoffatom oder eine geradkettige oder verzweigte C₁₋₄-Alkylgruppe und R₄ eine geradkettige oder verzweigte C₁₋₄-Alkylgruppe bedeuten;
- (d) der Verbindung der folgenden Formel (6): 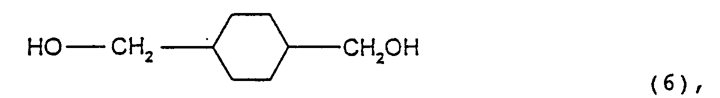
- (e) der Verbindung der folgenden Formel (7): 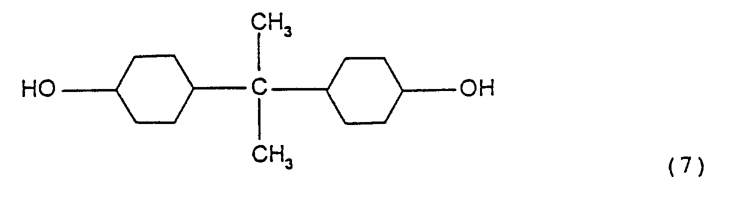 und
- (f) deren Gemischen.

12. Kosmetische oder dermatologische Zusammensetzung, die in einem kosmetisch oder dermatologisch akzeptablen Träger mindestens ein in dem Träger gelöstes oder dispergiertes filmbildendes Polymer und mindestens ein in einem der Ansprüche 1 bis 11 definiertes Oligomer, das in dem Träger gelöst oder dispergiert vorliegt, als Weichmacher für das filmbildende Polymer enthält, wobei die Zusammensetzung keine Nitrocellulose enthält.

13. Zusammensetzung nach Anspruch 12, dadurch gekennzeichnet, daß das weichmachende Oligomer in Konzentrationen von 0,1 bis 80 Gew.-% (Trockenmasse), bevorzugter von 5 bis 40 Gew.-% und insbesondere von 10 bis 30 Gew.-%, bezogen auf das Gesamtgewicht der Zusammensetzung, eingesetzt wird.

14. Zusammensetzung nach Anspruch 12 oder 13, dadurch gekennzeichnet, daß das filmbildende Polymer ausgewählt ist unter:
- den Vinylacetat/Crotonsäure/Vinyl-*tert*.-butylbenzoat-Copolymeren;
- den Acrylsäure/Ethylacrylat/N-*tert*.-Butylacrylamid-Copolymeren;
- den N-Ocytlacrylamid/Methylmethacrylat/Hydroxypropylmethacrylat/Acrylsäure/*tert*.-Butylaminoethylmethacrylat-Copolymeren und
- den Acrylsäure/Ethylacrylat/*tert*.-Butylacrylat-Terpolymeren.

15. Zusammensetzung nach einem der Ansprüche 12 bis 14, dadurch gekennzeichnet, daß der Mengenanteil des filmbildenden Polymers in den erfindungsgemäßen kosmetischen oder dermatologischen Zusammensetzungen im Bereich von 0,1 bis 50 Gew.-% und vorzugsweise von 1 bis 30 Gew.-%, bezogen auf das Gesamtgewicht der Zusammensetzung, liegt.

16. Zusammensetzung nach einem der Ansprüche 12 bis 15, dadurch gekennzeichnet, daß der kosmetisch oder dermatologisch akzeptable Träger aus Wasser, einem oder mehreren kosmetisch akzeptablen organischen Lösemitteln oder auch aus einem Gemisch von Wasser und einem oder mehreren kosmetisch akzeptablen organischen Lösemitteln oder auch aus Fettsubstanzen besteht, die ausgewählt sind unter Mineralölen, pflanzlichen, tierischen oder synthetischen Ölen, tierischen, fossilen, pflanzlichen, mineralischen oder synthetischen Wachsen.

17. Zusammensetzung nach Anspruch 16, worin die organischen Lösemittel niedere C₁₋₄-Alkohole oder Gemische von Butylacetat und Ethylacetat sind.

18. Zusammensetzung nach einem der Ansprüche 12 bis 17, dadurch gekennzeichnet, daß sie herkömmliche kosmetische Zusatzstoffe enthält, die ausgewählt sind unter Fettsubstanzen, wie Mineralölen, pflanzlichen, tierischen oder synthetischen Ölen, tierischen, fossilen, pflanzlichen, mineralischen oder synthetischen Wachsen, organischen Lösemitteln, Verdickungsmitteln, reizlindernden Mitteln, Mitteln gegen Schaumbildung, Hydratisierungsmitteln, Feuchthaltemitteln, Behandlungsmitteln, Antitranspirantien, Mitteln zum Alkalischmachen, Mitteln zum Ansäuren, UV-A-, UV-B- oder Breitband-Sonnenschutzfiltern, Färbemitteln, Pigmenten, Parfums, Konservierungsmitteln, anionischen, nichtionischen oder amphoteren organischen Polymeren und Treibmitteln.

19. Zusammensetzung nach einem der Ansprüche 12 bis 18, dadurch gekennzeichnet, daß es sich um eine Zusammensetzung für die Haare handelt.

20. Zusammensetzung nach einem der Ansprüche 12 bis 18, dadurch gekennzeichnet, daß es sich um eine Zusammensetzung zum Schminken handelt.

21. Zusammensetzung nach einem der Ansprüche 12 bis 18, dadurch gekennzeichnet, daß es sich um eine Zusammensetzung zur Pflege der Haut handelt.

22. Verfahren zur kosmetischen Behandlung von Keratinsubstanzen, dadurch gekennzeichnet, daß es darin besteht, eine in einem der Ansprüche 12 bis 21 definierte kosmetische Zusammensetzung auf die Keratinsubstanzen aufzubringen.
